# EUROPÄISCHE PATENTSCHRIFT

(11) **EP 1 170 025 B1**
(45) Veröffentlichungstag und Bekanntmachung des Hinweises auf die Patenterteilung: **24.08.2005**
(21) Anmeldenummer: 01810515.5
(22) Anmeldetag: 25.05.2001
(51) Int. Cl.: A61M 16/00, F04D 25/06, H02K 5/128

(54) **Gasfördervorrichtung für Beatmungs- und Narkosegeräte**
Gas supply device for ventilation and anaesthesia apparatus
Dispositif d'alimentation en gaz pour appareils de ventilation et anesthésie

(30) Priorität: 26.06.2000 EP 00810558
(43) Veröffentlichungstag der Anmeldung: 09.01.2002
(73) Patentinhaber: DRÄGERWERK AKTIENGESELLSCHAFT, 23558 Lübeck (DE)
(72) Erfinder: Schöb, Reto Dr., 8604 Volketswil (CH)

(56) Entgegenhaltungen:
- EP-A- 0 100 769
- WO-A-01/84690
- WO-A-97/10868
- WO-A-99/47197
- US-A- 3 107 310
- US-A- 5 906 203

## Beschreibung

Die Erfindung betrifft eine Gasfördervorrichtung für Beatmungs- und Narkosegeräte gemäss dem Oberbegriff von Anspruch 1.
Aus der Druckschrift WO 99/47 197 ist eine Gasfördervorrichtung für Beatmungsgeräte bekannt. Diese Gasfördervorrichtung umfasst alle Merkmale des Oberbegriffs von Anspruch 1.
Aus US 3,107,310 geht ein elektromagnetisch angetriebener Rotor mit einer axialen magnetischen Lagerung hervor. In WO 01/84 690 wird ein elektromagnetisches Antriebssystem für Flüssigkeitspumpen in Heizanlagen beschrieben.
Aufgabe der vorliegenden Erfindung ist die Verbesserung der bekannten, eingangs genannten Gasfördervorrichtung, um längere Betriebszeiten mit möglichst geringen Lagerwiderständen zu erzielen, wobei sich Druck- und Flussfluktuationen auf die Atemgasförderung praktisch nicht auswirken.
Diese Aufgabe wird gelöst mit einer Gasfördervorrichtung aufweisend die Merkmale von Anspruch 1. Die Unteransprüche 2 bis 31 betreffen weitere vorteilhafte Ausgestaltungen der erfindungsgemäßen Gasfördervorrichtung.
Die Aufgabe wird gelöst mit einer Gasfördervorrichtung für Beatmungs- und Narkosegeräte, umfassend einen Radialverdichter sowie eine Antriebsvorrichtung, welche die Lagerung und/oder das magnetische Drehfeld erzeugt, wobei der Radialverdichter ein Gehäuse umfasst, innerhalb welchem ein Verdichterrad und ein damit verbundener Rotor angeordnet ist, wobei die Antriebsvorrichtung für den Rotor außerhalb des Gehäuses angeordnet ist, und wobei der Rotor mit der Antriebsvorrichtung ein aktives Radiallager als Magnetlager ausbildet.
Die erfindungsgemäße Gasfördervorrichtung weist den Vorteil auf, dass der Radialverdichter sehr kostengünstig herstellbar ist, und dass das geförderte Fluid vollständig getrennt von der Antriebsvorrichtung ist. In einer bevorzugten Ausgestaltung sind der Radialverdichter und die Antriebsvorrichtung gegenseitig trennbar ausgestaltet. Dieser Radialverdichter ist vorteilhafterweise als Einwegprodukt beziehungsweise als Wegwerfprodukt ausgestaltet. Dies weist den Vorteil auf, dass der aufwändige Reinigungsprozess der Gasfördervorrichtung entfällt, da der Radialverdichter und vorteilhafterweise auch die übrigen, Fluid leitenden Komponenten als Einwegprodukte ausgestaltet sind. Somit ist zudem gewährleistet, dass eine neu verwendete Gasfördervorrichtung keimfrei ist.
Die Aufgabe wird zudem insbesondere gelöst mit einer Gasfördervorrichtung für Beatmungs- und. Narkosegeräte zusätzlich umfassend einen Bypass, wobei der Bypass eine Fluid leitende Verbindung zwischen der Saugseite und der Druckseite des Radialverdichters ausbildet. Dank diesem Bypass kann der Radialverdichter auch ohne Förderleistung betrieben werden, indem das Fluid über den Bypass umgewälzt wird, ohne dass der Radialverdichter bzw. der Antriebsmotor Schaden nimmt. In einer bevorzugten Ausführungsform ist am Bypass ein ansteuerbares Ventil angeordnet, welches den Querschnitt des Bypasses zu modulieren erlaubt, so dass der vom Radialverdichter erzeugte Fluiddruck über das Ventil steuerbar ist.
Die Aufgabe wird zudem insbesondere gelöst mit einer Gasfördervorrichtung für Beatmungs- und Narkosegeräte, wobei das Gehäuse und der Rotor derart bezüglich der separaten Antriebsvorrichtung angepasst ausgestaltet sind, dass der Rotor an die Antriebsvorrichtung koppelbar und von dieser antreibbar ist. Diese Anordnung weist den Vorteil auf, dass die Gasfördervorrichtung vollständig getrennt ist von der Antriebsvorrichtung. Die Gasfördervorrichtung kann an die Antriebsvorrichtung gekoppelt werden. Diese Gasfördervorrichtung ist vorzugsweise als Einwegprodukt ausgestaltet.
Die Erfindung wird nachfolgend mit Hilfe von mehreren Ausführungsbeispielen im Detail beschrieben.

Es zeigen:
- Fig. 1: Einen Längsschnitt durch eine Gasfördervorrichtung mit einem Radialverdichter und einem lagerlosen Elektromotor;
- Fig. 2: einen Längsschnitt durch ein erfindungsgemäßes Ausführungsbeispiel einer Gasfördervorrichtung mit einem Radialverdichter, zwei aktiven Radiallagern und einem Elektromotor;
- Fig. 3: einen Längsschnitt durch ein weiteres Ausführungsbeispiel einer Gasfördervorrichtung mit einem Radialverdichter, einem Spurlager sowie einem lagerlosen Elektromotor;
- Fig. 4: einen Längsschnitt durch ein weiteres Ausführungsbeispiel einer Gasfördervorrichtung mit einem Radialverdichter, einem Spurlager, einem passiven Radiallager sowie einem Elektromotor;
- Fig. 5: einen Querschnitt durch einen Radialverdichter mit einem geöffnetem Ventil;
- Fig. 6: einen Querschnitt durch einen Radialverdichter mit geschlossenem Ventil;
- Fig. 7: einen Querschnitt durch einen Radialverdichter mit einer weiteren Ausführungsform eines Ventils;
- Fig. 8: schematisch eine Gasfördervorrichtung mit einem Radialverdichter sowie weiteren am Radialverdichter angeschlossenen Komponenten;
- Fig. 9: einen Längsschnitt durch die Anordnung gemäss Fig. 1 mit einer zusätzlichen Zuleitung zur Befeuchtung der Luft;
- Fig. 10: eine schematische Darstellung der einzelnen Komponenten der Gasfördervorrichtung, welche für ein Beatmungsgerät angeordnet sind;
- Fig. 11: eine weitere schematische Darstellung der einzelnen Komponenten für ein Beatmungs- oder Narkosegerät;
- Fig. 12: schematisch die Anordnung der einzelnen Komponenten einer Gasfördervorrichtung, welche im wesentlichen als Einwegprodukt ausgestaltet ist;
- Fig. 13: einen Längsschnitt durch ein weiteres Ausführungsbeispiel einer Gasfördervorrichtung mit einem Radialverdichter, einem Spurlager und einem Elektromotor;.
- Fig. 14: einen Längsschnitt durch ein weiteres Ausführungsbeispiel einer Gasfördervorrichtung mit einem Radialverdichter, einem Spurlager und einer Magnetkupplung.

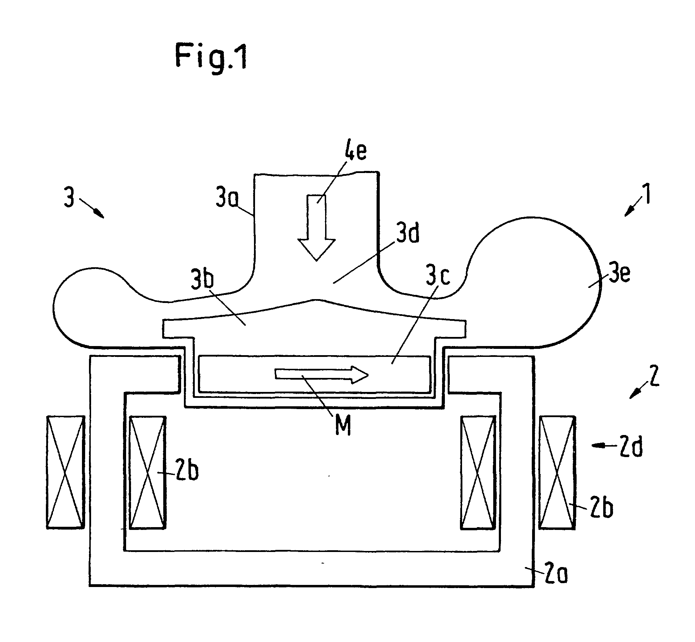

Figur 1 zeigt eine Gasfördervorrichtung 1 umfassend eine Antriebsvorrichtung 2 sowie einen Radialverdichter 3. Der Radialverdichter 3 umfasst ein Gehäuse 3a innerhalb welchem ein Verdichterrad 3b mit nicht dargestellten Schaufeln angeordnet ist, wobei in dem aus Kunststoff bestehenden Verdichterrad 3b zudem ein als Permanentmagnet ausgestalteter Motorrotor 3c angegossen ist, welcher eine Magnetisierung M aufweist. Der Radialverdichter 3 weist einen Gaseinlass 3d bzw. eine Saugseite 3d sowie einen Gasauslass 3e bzw. eine Druckseite 3e auf, sodass während dem Betrieb des Radialverdichters 3 ein Gasstrom 4e entsteht. Der Radialverdichter 3 ist lösbar mit dem darunter angeordneten Motorstator 2d verbunden. Der Motorstator 2d bildet zusammen mit dem Motorrotor 3c einen Elektromotor aus, wobei der Elektromotor als ein lagerloser Motor ausgestaltet ist. Ein derartiger, lagerloser Motor ist beispielsweise in der Druckschrift EP 0 819 330 offenbart. Der Motorstator 2d umfasst ein Blechpaket aus Weicheisen 2a, in dessen Nuten eine Motorwicklung eingelegt ist, welche derart von einer nicht dargestellten Ansteuervorrichtung ansteuerbar ist, dass ein Drehfeld oder ein Wechselfeld mit einer ersten Polpaarzahl p erzeugt wird, sodass der Permanentmagnet 3c, welcher in Umfangsrichtung ebenfalls mit der Polpaarzahl p magnetisiert ist, und damit auch das Verdichterrad 3b in Umfangsrichtung angetrieben wird. Die Motorwicklung 2b ist im Weiteren derart ausgestaltet, dass sich zusätzlich ein Drehfeld mit einer zweiten Polpaarzahl p+1 oder p-1 ausbildet. Dieses Drechfeld wird derart geregelt, dass der Permanentmagnet 3c mit Verdichterrad 3b in radialer Richtung berührungslos gehalten wird. In axialer Richtung sowie gegen Verkippen wird der Permanentmagnet 3c und damit auch das Verdichterrad 3b durch passive Magnetkräfte in einer stabilen Lage gehalten. Wie in Figur 1 angedeutet, kann der Radialverdichter 3 auf einfache Weise in den Motorstator 2d eingesetzt werden und auch wieder davon abgenommen werden. Dies wird dadurch erreicht, dass der Motorstator 2d als sogenannter Tempel-Motorstator ausgeführt wird. In einer bevorzugten Ausgestaltung ist das Gehäuse 3a sowie das Verdichterrad 3b aus einem Kunststoff ausgestaltet und als Wegwerf- bzw. als Einwegprodukt konzipiert. Diese erfindungsgemässe Anordnung ermöglicht, dass der Motorstator 2d wieder verwendbar ist, wogegen der Radialverdichter 3 vorzugsweise nur ein einziges Mal verwendet und danach entsorgt wird. Dieser Radialverdichter 3 ist sehr kostengünstig herstellbar, da dieser in einer einfachen Ausführungsform, ausser dem ebenfalls sehr kostengünstigen Permanentmagnet 3c, nur noch das Gehäuse 3a und das Verdichterrad 3b umfasst.

Diese Anordnung weist insbesondere den Vorteil auf, dass der Radialverdichter 3 als Wegwerfprodukt konzipiert ist und somit keiner Reinigung bedarf, sodass bei jedem Einsatz des Beatmungs- oder Narkosegerätes die Gewissheit besteht, dass der Radialverdichter 3 zu Beginn in einem keimfreien Zustand ist.

Die Gasfördervorrichtung 1 weist den zusätzlichen Vorteil auf, dass das Verdichterrad 3b eine sehr geringe Masse aufweist, und dass das Verdichterrad 3b berührungslos im Radialverdichter 3 gehalten ist, durch die als lagerloser Motor ausgestaltete Antriebsvorrichtung 2. Dies ermöglicht einerseits eine sehr hohe Drehzahl des Verdichterrades 3b als auch eine schnelle Drehzahlvariation des Verdichterrades 3b. Dies erlaubt, über eine entsprechende Ansteuerung der Motorwicklungen 2b, innerhalb weniger Millisekunden eine grosse Drehzahl bzw. Druckänderung zu erreichen. Somit können Druckänderung bzw. Atemmuster durch eine entsprechende Modulation der Drehzahl des Verdichterrades 3b erzeugt werden.

Ein weiterer Vorteil des dargestellten Motors ist die Tatsache, dass das Verdichterrad 3b eine geringe Masse aufweist, dass das Verdichterrad 3b berührungslos gelagert ist, und dass daher zu dessen Betrieb eine geringe elektrische Leistung erforderlich ist. Dies erlaubt die erfindungsgemässe Gasfördervorrichtung auch mit einer Batterie zu betreiben und somit als eine portable bzw. netzunabhängige Gasfördervorrichtung 1 auszugestalten. Dies ist insbesondere bei der Verwendung für Beatmungsgeräte von zentraler Bedeutung, um die Gasfördereinrichtung 1 als portables Gerät zu verwenden oder zum Beispiel im Heimbereich als kleines, handliches Gerät zu nutzen.

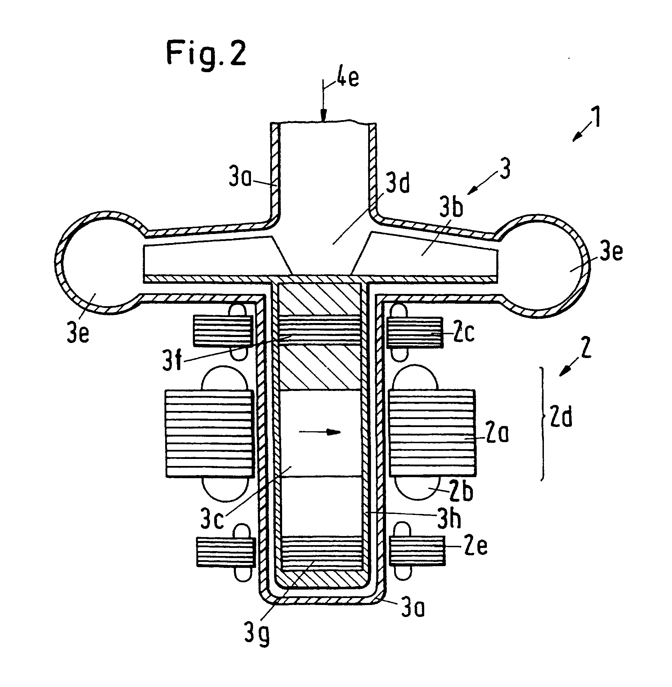

Figur 2 zeigt eine erfindungsgemäße Gasfördervorrichtung 1 mit einer Antriebsvorrichtung 2 und einem berührungslos gelagerten und angetriebenen Radialverdichter 3. Innerhalb des Gehäuses 3a ist das Verdichterrad 3b über einen zapfenartigen Fortsatz mit einem Permanentmagnet 3c sowie zwei Magnetlagerrotoren 3f, 3g verbunden. Die beiden vertikal versetzt angeordneten Radialmagnetlagerstatoren 2c, 2e bilden zusammen mit den Magnetlagerrotoren 3f, 3g je ein aktives Radiallager aus. Die Magnetlagerrotoren 3f, 3g sind als ein Packet Blechscheiben ausgestaltet. Der Motorstator 2d mit Motorenwicklungen 2b und Blechpaket 2a bildet zusammen mit dem Motorrotor 3c einen Elektromotor, welcher das Verdichterrad 3b antreibt. Die auftretenden Reluktanzkräfte üben in vertikaler Richtung eine passiv wirkende Kraft aus, sodass das Verdichterrad 3b auch in vertikaler Richtung berührungslos gehalten wird.

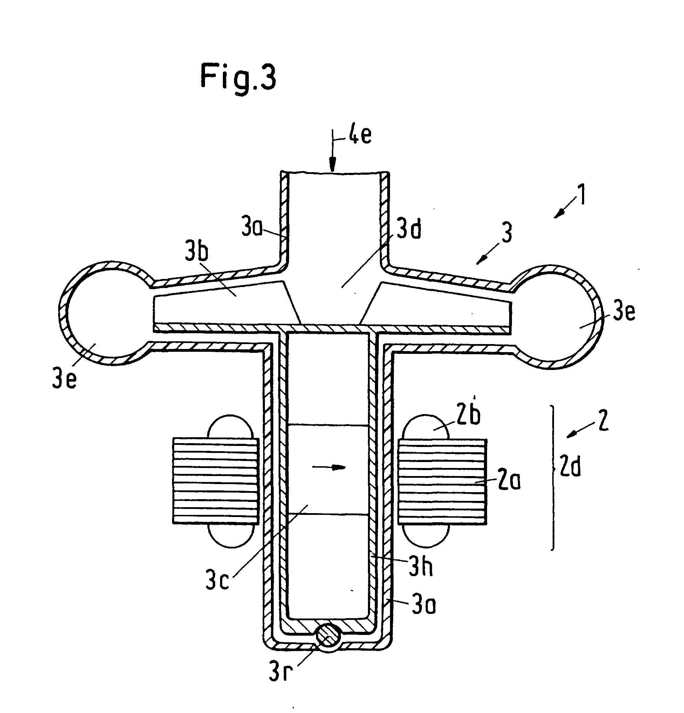

Figur 3 zeigt ein weiteres Ausführungsbeispiel einer Gasfördervorrichtung 1. Sie umfasst eine Antriebsvorrichtung 2 und einen Radialverdichter 3. Im Unterschied zu dem in Figur 2 dargestellten Ausführungsbeispiel umfasst die Antriebsvorrichtung 2 nur den lagerlosen Elektromotor mit Motorrotor 3c und Motorstator 2d, wobei im Motorstator 2d durch ein entsprechendes Ansteuern der Spulen 2b ein magnetisches Drehfeld erzeugt, um den Permanentmagnet-Läufer 3c in Rotation zu versetzen sowie radial, magnetisch zu lagern. Natürlich kann der Motorrotor auch als Induktionsmotorläufer oder als Reluktanzmotorläufer ausgeführt sein. In vertikaler Richtung ist das Verdichterrad 3b durch ein unten im Gehäuse 3a angeordnetes Spurlager 3r in vertikaler Richtung abgestützt und geführt. Dadurch wird der Rotor sowohl in axialer Richtung als auch gegen Verkippen stabilisiert. Der Motorstator 2d ist gegenüber dem Motorrotor 3c leicht nach unten versetzt, um eine geringe, vertikal nach unten gerichtete Magnetkraft zu erzeugen.

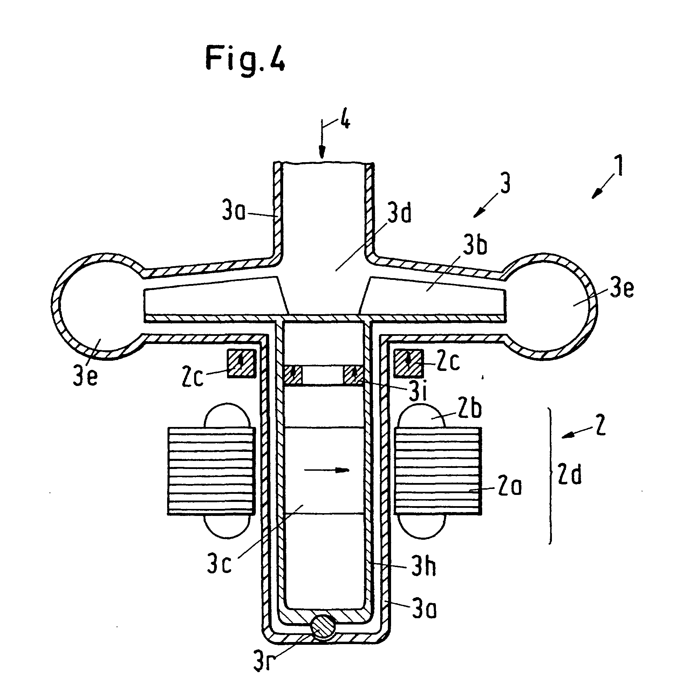

Zusätzlich zu der in Figur 3 dargestellten Ausführungsform weist die in Figur 4 dargestellte Gasfördervorrichtung 1 ein passives Radialmagnetlager auf, umfassend einen ausserhalb des Gehäuses 3a angeordneten Permanentmagnetring 2c sowie einen mit dem Verdichterrad 2b fest verbundenen Permanentmagnetring 3i. Da das passive Radialmagnetlager bereits eine radiale Führung des Rotors 3c bewirkt, kann anstelle eines lagerlosen Elektromotors ein konventioneller Elektromotor eingesetzt werden, welcher den Rotor 3c nur antreibt. Dadurch kann insbesondere die Ansteuerelektronik für den Motorstator 2d erheblich vereinfacht werden. Der ausserhalb des Gehäuses 3a angeordnete Permanentmagnetring 2c, welcher den Magnetlagerstator des passiven Radialmagnetlagers bildet, ist gegenüber dem mit dem Verdichterrad 2b fest verbundenen Permanentmagnetring 3i, welcher den Magnetlagerrotor bildet, vertikal leicht nach oben versetzt angeordnet. Dadurch wirkt eine geringe, vertikal nach unten gerichtete Magnetkraft auf den Rotor, welche vom Spurlager 3r aufgenommen wird. Der Rotor wird somit axial in beiden Richtungen festgehalten.

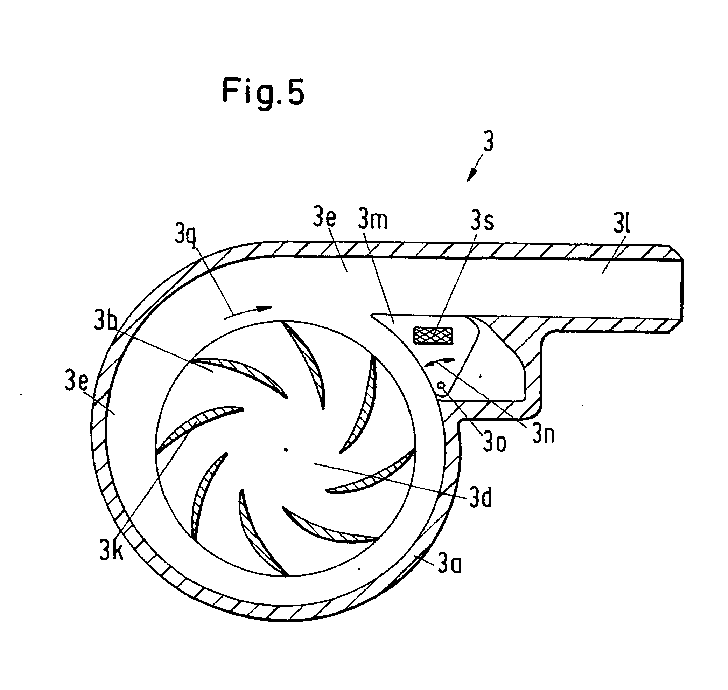

Fig. 5 zeigt einen Querschnitt durch einen Radialverdichter 3. Innerhalb des Gehäuses 3a ist ein Verdichterrad 3b mit Schaufeln 3k in Drehrichtung 3q drehbar gelagert. Das geförderte Fluid gelangt über den Gaseinlass 3d bzw. die Saugseite 3d in das Verdichterrad 3b und wird nachfolgend zum Gasauslass 3e beziehungsweise zur Druckseite 3e gefördert und gelangt danach zum Gasaustritt 31. Vor dem Gasaustritt 31 ist ein zungenförmig ausgestaltetes Ventil 3m angeordnet, welches um ein Drehzentrum 3o in Bewegungsrichtung 3n drehbar gelagert ist. In der Zunge 3m ist zudem ein Permanentmagnet 3s angeordnet.

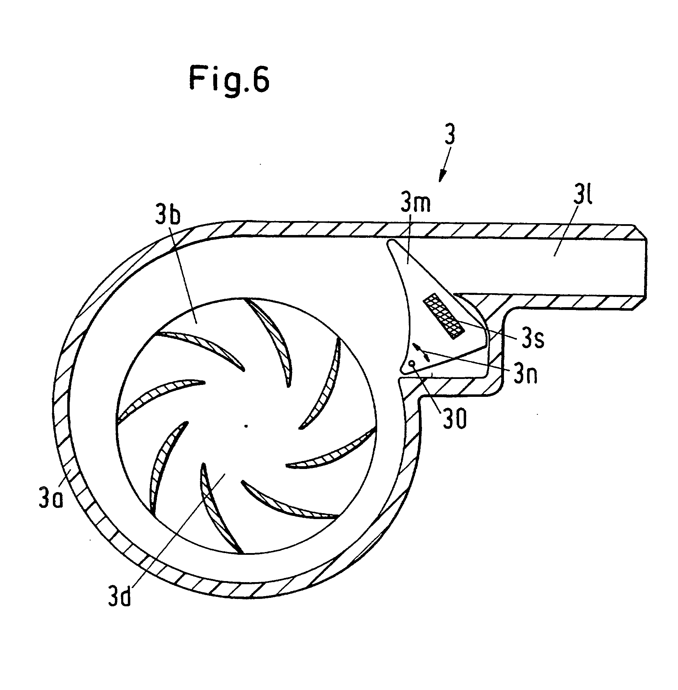

Fig. 6 zeigt einen Querschnitt durch einen Radialverdichter 3 mit geschlossenem Ventil 3m. In dieser Stellung wird das sich im Radialverdichter 3 befindliche Fluid umgewälzt ohne dabei in den Gasaustritt 31 zu gelangen. Ausserhalb des Gehäuses 3a ist ein nicht dargestellter, beweglicher Permanentmagnet angeordnet, welcher auf den Permanentmagnet 3s der Zunge 3m einwirkt, sodass die Zunge 3m berührungslos bewegbar ist. Die Position des ausserhalb des Gehäuses angeordneten Permanentmagneten wird über ein Stellglied geregelt. Dadurch lässt sich die Stellung des Ventils 3m steuern und damit der Gasdruck am Gasaustritt 3l beziehungsweise der Volumenstrom beeinflussen. In offener Ventilstellung, gezeigt in Figur 5, ist der Gasdruck beziehungsweise der Volumenstrom maximal und wird bei einer gegebenen Drehzahl durch die Drosselkurve des Gebläses sowie durch den Ströhmungswiderstand des ganzen Beatmungssystems inklusive Katheter, Luftröhre, Lunge usw. bestimmt. In geschlossener Stellung, gezeigt in Figur 6, ist der Gasdruck am Gasaustritt 31 sowie der Volumenstrom minimal, in der Praxis beinahe 0. Durch Variation der Ventilstellung lässt sich somit der Gasdruck am Gasaustritt 31 beziehungsweise der Volumenstrom einstellen. Durch zeitliche Änderung der Ventilstellung lassen sich fast beliebige Beatmungsmuster erzeugen. In Kombination mit einem Drucksensor beziehungsweise einem Volumenstromsensor lassen sich Druckverläufe beziehungsweise Volumenstromverläufe sehr genau regeln.

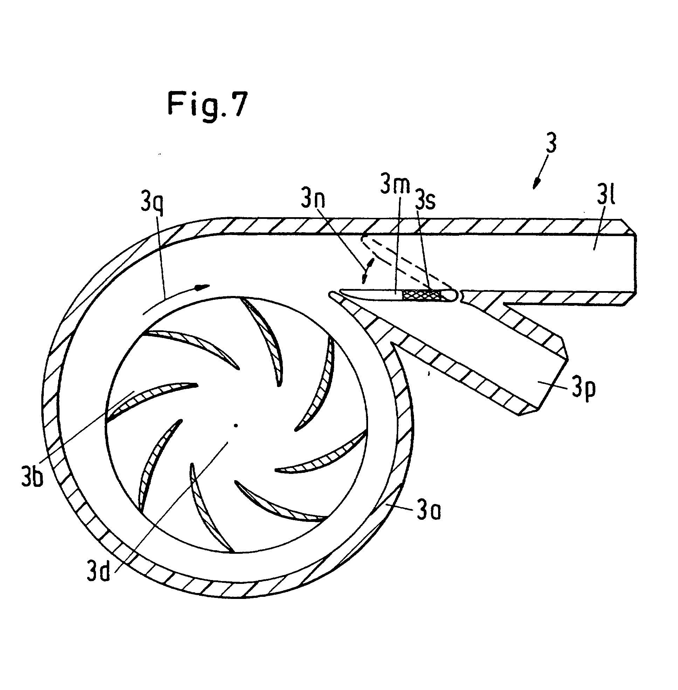

Fig. 7 zeigt einen Querschnitt durch ein weiteres Ausführungsbeispiel eines Radialverdichters 3. Dieser Radialverdichter 3 weist zwei Gasaustritte 31, 3p auf, wobei die in Bewegungsrichtung 3n schwenkbar gelagerte Zunge 3m mit Permanentmagnet 3s das vom Radialverdichter 3 geförderte Fluid ansteuerbar zumindest teilweise dem Gasaustritt 31 und/oder dem Gasaustritt 3p zuführt. Der Gasaustritt 31 wird beispielsweise über ein Schlauchsystem mit einer Beatmungsmaske oder einem Beatmungskatherer verbunden, während der Gasaustritt 3p über einen Bypassschlauch zum Gaseinlass 3d zurückgeführt wird oder über einen Bakterienfilter an die Umgebungsluft geleitet wird. Durch Variation der Ventilstellung lassen sich wiederum fast beliebige Beatmungsmuster erzeugen.

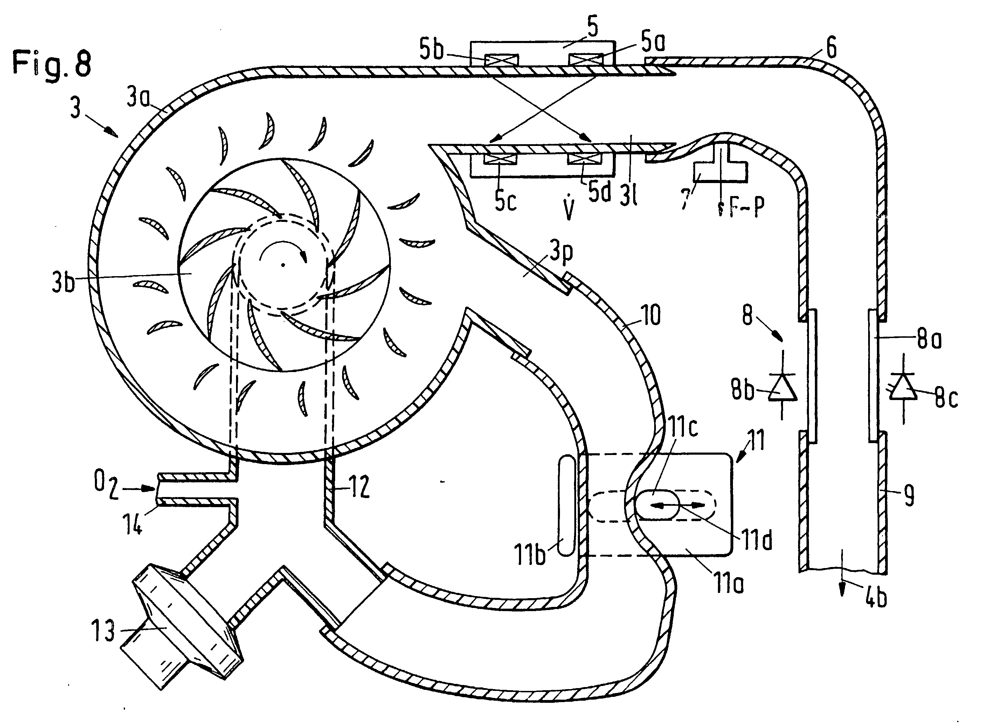

Fig. 8 zeigt eine Gasfördervorrichtung 1 umfassend einen Radialverdichter 3 sowie eine Mehrzahl daran angeschlossener Komponenten. Der Radialverdichter 3 weist einen ersten Gasaustritt 31 auf, um welchen eine Volumenstrommessvorrichtung 5 mit zwei Ultraschallsendern 5a, 5b und zwei Ultraschallempfängern 5c, 5d angeordnet ist, um den Volumenstrom des den Gasaustritt 31 durchströmenden Fluides zu messen. Dem ersten Gasaustritt 31 nachgeordnet ist ein elastischer Schlauch 6 angeordnet, welcher in ein lichtdurchlässiges Rohr 8a mündet, wobei nachfolgend wiederum ein elastischer Schlauch 9 angeordnet ist. Beim elastischen Schlauch 6 ist eine Druckmessvorrichtung 7 angeordnet, welche einen Drucksensor aufweist, um den Druck P des sich innerhalb des elastischen Schlauchs 6 befindlichen Fluides zu messen. Das lichtdurchlässige Rohr 8a bildet Teil eines Gassensors 8 umfassend einen Fotosensor 8b mit Filter und eine Lichtquelle 8c, welche beispielsweise als LED oder als eine Laserdiode ausgestaltet ist. Die drei vorhin genannten Sensoren 5, 7 und 8 erlauben somit den Volumenstrom, den Druck sowie die Gaszusammensetzung des geförderten Fluides zu messen.

Der Radialverdichter 3 umfasst einen zweiten Gasaustritt 3p, welcher über einen elastischen Schlauch 10 in die Leitung 12 und darauf folgend wieder in den Gaseinlass 3d des Radialverdichters 3 mündet. Der elastische Schlauch 10 bildet somit einen Bypass aus. In einer bevorzugten Ausgestaltung ist die Menge des durch den Bypass 10 strömenden Fluides regelbar. Im dargestellten Ausführungsbeispiel ist am Bypass 10 ein Bypassregelventil 11 angeordnet, welches als Linearaktor 11 a ausgestaltet ist, mit einer Schlauchführung 11 b und einem in Bewegungsrichtung 11 d linearbeweglichen Schliessstück 11c, welches den elastischen Schlauch 10 zu quetschen erlaubt, was den Querschnitt des elastischen Schlauches 10 verringert. An der Leitung 12 ist zudem ein Bakterienfilter 13 für Frischluft angeordnet sowie eine Sauerstoffzuleitung 14.

Die in Fig. 8 dargestellte Ausführungsform weist die Eigenschaft auf, dass langsame Druckschwankänderungen beispielsweise mit einer Slew-Rate von weniger als 200 Millibar pro Sekunde durch eine entsprechende Drehzahlvariation des Verdichterrades 3b erzeugbar sind. Schnelle Druckschwankungen mit einer Slew-Rate von mehr als 200 Millibar pro Sekunde werden vorzugsweise dadurch erzeugt, dass das Bypassregelventil 11 entsprechend angesteuert wird. Durch Kombination von Drehzahlvariation und Einstellung des Bypassregelventils lassen sich fast alle bekannten Beatmungsmuster erzeugen. Insbesondere können in Kombination mit einem Drucksensor 7 und/oder einem Volumenstromsensor 5 Druckprofile beziehungsweise Volumenstromprofile genau geregelt werden. Ein weiterer Vorteil der in Fig. 8 dargestellten Ausführungsform ist darin zu sehen, dass alle Komponenten, innerhalb welcher das Fluid gefördert wird, welche beispielsweise den Radialverdichter 3, den elastischen Schlauch 6, das lichtdurchlässige Rohr 8a, den elastischen Schlauch 9, den Bypass 10 sowie die Leitung 12 mit Frischluftfilter 13 umfassen, aus einem Kunststoff herstellbar sind, und dass all diese Komponenten keine teuren Gegenstände wie Sensoren aufweisen. In einer bevorzugten Ausgestaltung sind diese Fluid leitenden Komponenten daher als Einweg- bzw. als Wegwerfprodukt konzipiert. Diese Komponenten sind derart ausgestaltet, dass der Radialverdichter 3 auf einfache Weise in den Motorstator 2 einführbar ist, und dass die übrigen Komponenten auf einfache Weise in die Volumenstrommessvorrichtung 5, die Druckmessvorrichtung 7, den Gasbestimmungssensor 8 sowie das Bypassregelventil 11 einlegbar sind. Diese teuren Vorrichtungen, Sensoren und Ventile können wieder verwendet werden, wobei diese zudem vor einer bakteriologischen Verunreinigung geschützt sind, weil das aus Kunststoff bestehende, Fluid leitende System gegen Aussen abgedichtet ist.

Ein weiterer Vorteil der in Fig. 8 dargestellten Gasfördervorrichtung 1 ist darin zu sehen, dass vom elastischen Schlauch 9 her ein sogenanntes "Durchhusten" möglich ist, das heisst, dass ständig eine Fluid leitende Verbindung mit geringem Strömungswiederstand zwischen dem elastischen Schlauch 9 und einem Auslass, im dargestellten Beispiel dem Filter 13, besteht. Die von einem Patienten durch ein Husten fast schlagartig erzeugte Gasvolumenänderung wird innerhalb der Gasfördervorrichtung 1 nicht wesentlich behindert, kann sich durch das Gebläse fortpflanzen, und entspannt sich über das Filter 13 ohne dass es zu einem schmerzhaften oder gar gefährlichen Druckanstieg in der Lunge kommt..

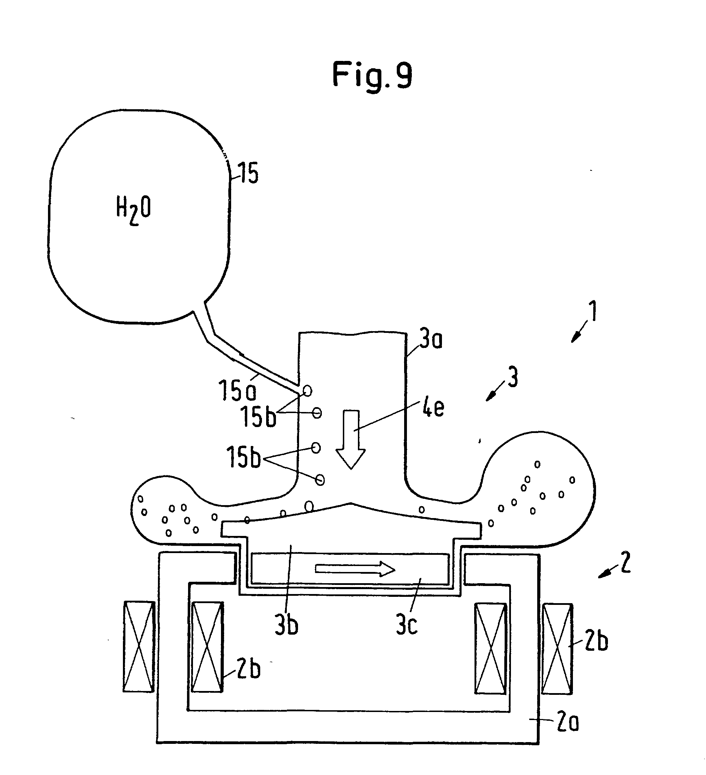

Zusätzlich zu der in Fig. 1 dargestellten Gasfördervorrichtung 1 umfasst das in Fig. 9 dargestellte Ausführungsbeispiel einen Flüssigkeitsbehälter 15, insbesondere für Wasser, wobei der Flüssigkeitsbehälter 15 über eine Zuleitung 15a oberhalb des Verdichterrades 3b in das Gehäuse 3a des Radialverdichters 3 mündet. Die sich innerhalb des Gehäuses 3a bildenden Flüssigkeitstropfen 15b fallen auf das mit einer hohen Drehzahl von beispielsweise 30'000 Umdrehungen pro Minute rotierende Verdichterrad 3b, wobei die Flüssigkeitstropfen 15b aufgrund der hohen Drehzahl auf feinste Art vernebelt werden, sodass der Gasstrom 4e im Radialverdichter 3 befeuchtet wird.

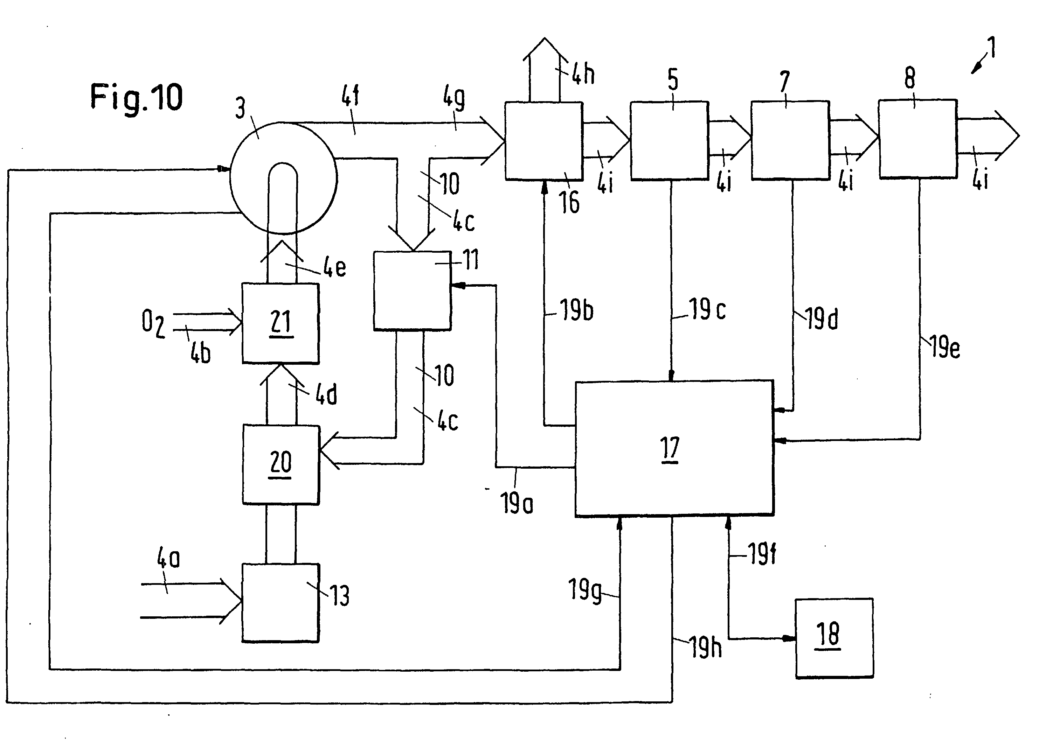

Fig. 10 zeigt ein umfassendes Ausführungsbeispiel einer Gasfördervorrichtung 1, welche für ein Beatmungsgerät- oder Narkosegerät geeignet ist. Die Frischluft 4a wird über ein Filter 13 angesogen und gelangt über das Speichervolumen 20 als Gasstrom 4d in das Kupplungsstück 21, in welchem zudem ein Sauerstoffgasstrom 4b,andere Beatmungsgase oder Narkosegase beigemischt werden. Diese Mischung wird als Gasstrom 4e vom Radialverdichter 3 angesogen, und als Gasstrom 4f weitergefördert, wobei ein Teil dieses Gasstroms als Bypassgasstrom 4c über den Bypass 10 und das Bypassregelventil 11 dem Speichervolumen 20 zugeleitet wird, wogegen der andere Anteil des Gasstroms 4f als Gasstrom 4g bzw. 4i dem Patienten zur Unterstützung der Atmung zugeführt wird. Im Ausatmungsventil 16 ist ein über die Steuervorrichtung 17 schaltbares Ventil angeordnet, welches beim Ausatmen den Fluidstrom 4i derart umsteuert, dass ein Ausatmungsgasstrom 4h entsteht. Dem Ausatmungsventil 16 ist eine Volumenstrommessvorrichtung 5, nachfolgend eine Druckmessvorrichtung 7 und nachfolgend ein Gasbestimmungssensor 8 nachgeordnet. Das Bypassregelventil 11, das Ausatmungsventil 16, die Volumenstrommessvorrichtung 5, die Druckmessvorrichtung 7 sowie der Gasbestimmungssensor 8 sind über elektrische Leitungen 19a, 19b, 19c, 19d, 19e signalübertragend mit der Steuervorrichtung 17 verbunden. Zudem ist eine Ein- / Ausgabevorrichtung 18 angeordnet, welche ebenfalls über eine elektrische Leitung 19f mit der Steuervorrichtung 17 verbunden ist. Die Steuervorrichtung 17 ist zudem über elektrische Leitungen 19g, 19h mit dem Radialverdichter 3 verbunden, um dessen Drehzahl zu regeln und dessen Drehzahl bzw. dessen Zustand zu messen oder zu überwachen.

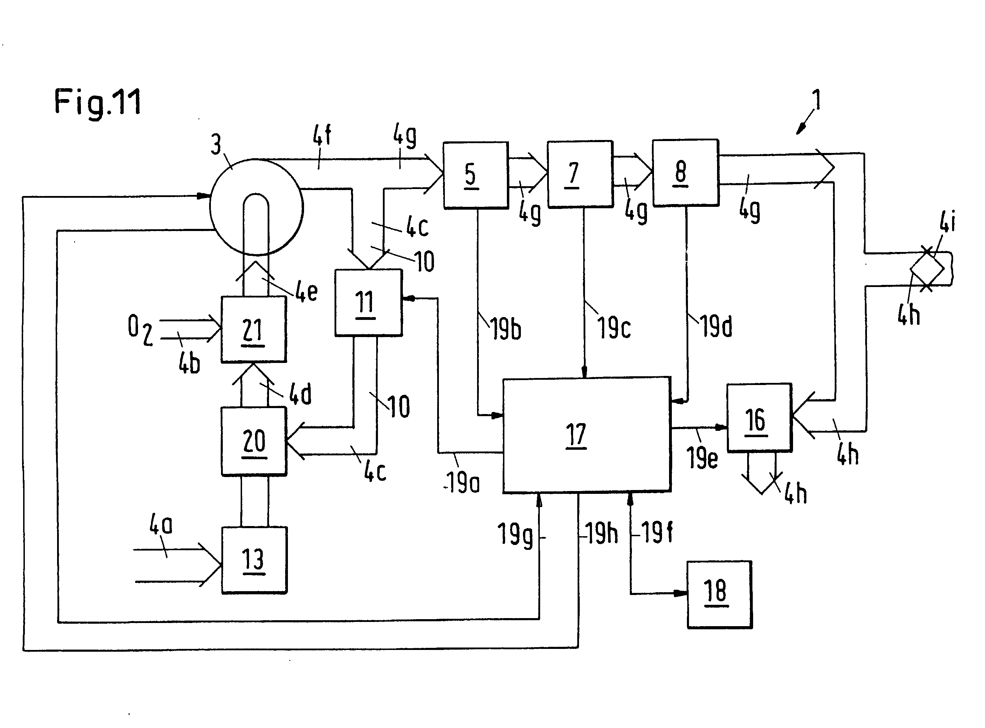

Fig. 11 zeigt in einer schematischen Darstellung eine weitere Gasfördervorrichtung 1 ausgestaltet zur Verwendung als Beatmungs- oder Narkosegerät. Im Unterschied zu der in Fig. 10 dargestellten Ausführungsform ist in der Gasfördervorrichtung 1 gemäss Fig. 11 das Ausatmungsventil 16 nicht im Gasstrom 4g, sondern in einem getrennten Pfad angeordnet, sodass der Ausatmungsgasstrom 4h über das Ausatmungsventil 16 geleitet wird, wobei das Ausatmungsventil 16 derart von der Steuervorrichtung 17 angesteuert wird, dass es während der Ausatmungsphase des Patienten geöffnet ist.

In einer bevorzugten Ausführungsform werden bei den in Fig. 10 und 11 dargestellten Gasfördervorrichtungen 1 die Fluid leitenden Verbindungsmittel als Einweg- bzw. als Wegwerfprodukte ausgestaltet, wobei diese Wegwerfprodukte vorzugsweise auch den Radialverdichter 3 umfassen. Diese wegwerfbaren Komponenten sind derart ausgestaltet, dass sie in die teureren Komponenten wie das Bypassregelventil 11, die Volumenstrommessvorrichtung 5, die Druckmessvorrichtung 7 oder den Gasbestimmungssensor 8 oder das Ausatmungsventil 16 einlegbar sind.

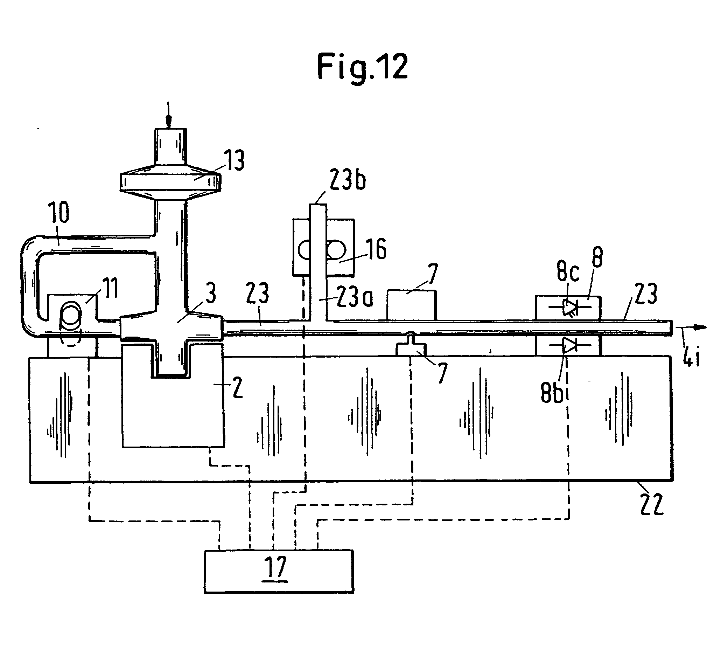

Fig. 12 zeigt schematisch eine derartige Anordnung, wobei der Gasverdichter 3, der Filter 13, der elastische Schlauch 10, der elastische Schlauch 23 und die Abzweigung 23a zusammenhängend ausgestaltet sind, und beispielsweise auch einstückig ausgestaltet sind. Diese Elemente können auf sehr einfache Weise in das Bypassregelventil 11, in den Motorstator 2, in das Ausatmungsventil 16, die Druckmessvorrichtung 7 und den Gasbestimmungssensor 8 eingelegt werden. Nach diesem Einlegen ist die Gasfördervorrichtung 1 betriebsbereit und die einzeln ansteuerbaren Komponenten über die Steuervorrichtung 17 ansteuerbar. Ein wesentlicher Vorteil der in Fig. 12 dargestellten Anordnung ist darin zu sehen, dass die Fluid leitendend Komponenten sehr günstig herstellbar sind und daher als Einweg- bzw. Wegwerfprodukte konzipierbar sind. Es ist daher nicht mehr erforderlich die Fluid leitenden Komponenten in einem aufwendigen Reinigungsprozess zu entkeimen. Bei jedem neuen Einsatz einer Fluid leitenden Komponente ist sicher gestellt, dass diese keimfrei ist, was insbesondere beim Intubieren eines Patienten das Risiko einer Pneumonie erheblich reduziert.

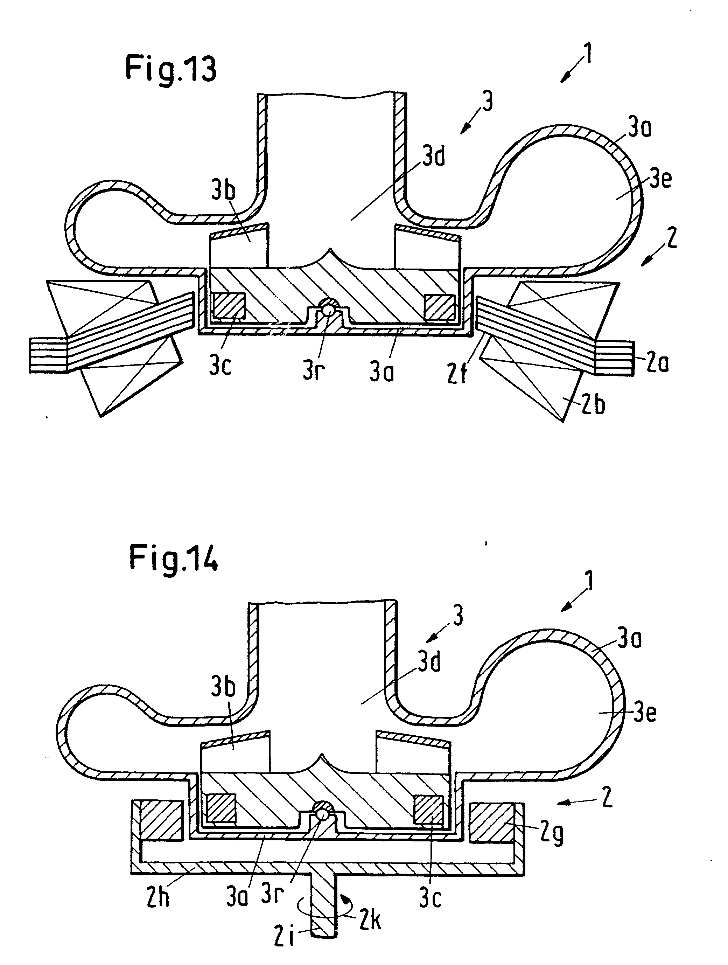

Fig. 13 zeigt in einem Längsschnitt ein weiteres Ausführungsbeispiel einer Gasfördervorrichtung 1. Innerhalb des Radialverdichters 3 ist ein auf einem Spurlager 3r gelagertes Verdichterrad 3b angeordnet. Das Verdichterrad 3b besteht aus Kunststoff. Innerhalb des Verdichterrades 3b ist ein Permanentmagnetring 3c oder einer Mehrzahl von Permanentmagnetsegmenten angeordnet. Der Motorstator 2 umfasst ein Blechpaket 2a mit Motorwicklungen 2b und Statorzähnen 2f. Der Motorstator 2 ist derart bezüglich dem Motorrotor 3c angeordnet und ausgestaltet, dass das Verdichterrad 3b in radialer Richtung sowie in axialer Richtung gegen unten durch das Spurlager 3r gelagert ist, und in axialer Richtung gegen oben und gegen eine Verkippung passivmagnetisch gelagert ist.

Fig. 14 zeigt in einem Längsschnitt ein weiteres Ausführungsbeispiel einer Gasfördervorrichtung 1 mit einem Radialverdichter 3 und einer als Magnetkupplung ausgestalteten Antriebsvorrichtung 2. Das Verdichterrad 3b ist in diesem Ausführungsbeispiel wie in Fig. 13 über ein Spurlager 3r gelagert. Zum Antrieb des Verdichterrades 3b bzw. des als Permanentmagnet ausgestalteten Motorrotors 3c ist eine kreisförmige, das Verdichterrad 3b in Umfangrichtung umschliessende Magnetkupplung 2g angeordnet, welche in Drehrichtung 2k von einer nicht dargestellten Antriebsvorrichtung angetrieben und über die Welle 2i mit der Antriebsvorrichtung verbunden ist. Die Welle 2i mündet in eine kreisförmige Kupplung 2h, an welcher ein ringförmiger Antriebsmagnet 2g angeordnet ist. Das Antriebsmagnet 2g kann auch aus einer Mehrzahl von Permanentmagnetsegmenten bestehen. Die Magnetkupplung 2g ist derart bezüglich dem Motorrotor 3c angeordnet und ausgestaltet, dass das Verdichterrad 3b in radialer Richtung sowie in axialer Richtung gegen unten durch das Spurlager 3r gelagert ist, und in axialer Richtung gegen oben sowie gegen ein Verkippen durch die passiven Magnetkräfte gelagert ist.

## Patentansprüche

1. Gasfördervorrichtung (1) für Beatmungs- und Narkosegeräte, umfassend einen Radialverdichter (3) sowie eine ein magnetisches Drehfeld erzeugende Antriebsvorrichtung (2), wobei der Radialverdichter (3) ein Gehäuse (3a) umfasst, innerhalb weichem ein Verdichterrad (3b) und ein damit verbundener Rotor (3c,3f,3g) angeordnet ist, wobei die Antriebsvorrichtung (2) für den Rotor (3c,3f,3g) außerhalb des Gehäuses (3a) angeordnet ist,
**dadurch gekennzeichnet, dass**
der Rotor (3c,3f,3g) mit der Antriebsvorrichtung (2) ein aktives Radiallager (2c, 3f; 2e, 3g) als Magnetlager ausbildet.

2. Gasfördervorrichtung (1) nach Anspruch 1, **dadurch gekennzeichnet, dass** innerhalb des Gehäuses (3a) ein Spurlager (3r) angeordnet ist, welches das Verdichterrad (3b) und den Rotor (3c) bezüglich des Gehäuses (3a) drehbar lagert.

3. Gasfördervorrichtung (1) nach einem der vorhergehenden Ansprüche, **dadurch gekennzeichnet, dass** die Antriebsvorrichtung (2) als Motorstator (2d) umfassend eine Motorwicklung (2b) und einen Eisenrückschluss (2a) ausgebildet ist, so dass der Motorstator (2d) den Rotor (3c) antreibt.

4. Gasfördervorrichtung (1) nach Anspruch 1, **dadurch gekennzeichnet, dass** der Motorstator (2a; 2d) zusammen mit dem Rotor (3c) als lagerloser Motor ausgestaltet ist.

5. Gasfördervorrichtung (1) nach einem der vorhergehenden Ansprüche, **dadurch gekennzeichnet, dass** der Rotor (3c) einen Permanentmagneten umfasst.

6. Gasfördervorrichtung (1) nach einem der Ansprüche 1 bis 3, **dadurch gekennzeichnet, dass** die Antriebsvorrichtung (2) eine drehbare Magnetkupplung (2g) umfasst, welche derart bezüglich des Rotors (3c) angeordnet werden kann, dass die drehende Magnetkupplung (2g) den Rotor (3c) antreibt.

7. Gasfördervorrichtung (1) nach einem der vorhergehenden Ansprüche, **dadurch gekennzeichnet, dass** der Radialverdichter (3) und die Antriebsvorrichtung (2) gegenseitig trennbar ausgestaltet sind.

8. Gasfördervorrichtung (1) nach einem der vorhergehenden Ansprüche, **dadurch gekennzeichnet, dass** der Radialverdichter (3) im Bereich des Gasaustritts (31) ein Ventil (3m), insbesondere ausgestaltet als eine verstellbare Zunge, aufweist, um den Gasfluss zumindest teilweise zu unterbrechen.

9. Gasfördervorrichtung (1) nach Anspruch 8, **dadurch gekennzeichnet, dass** das Ventil (3m) schwenkbar gelagert ist und insbesondere um ein Drehzentrum (3o) beweglich gelagert ist.

10. Gasfördervorrichtung (1) nach einem der Ansprüche 8 oder 9, **dadurch gekennzeichnet, dass** das Ventil (3m) einen Permanentmagneten (3s) umfasst, und dass außerhalb des Radialverdichters (3) eine entsprechend angepasst ausgestaltete Antriebsvorrichtung angeordnet ist, um das Ventil (3m) über den Permanentmagneten (3s) zu bewegen.

11. Gasfördervorrichtung (1) nach einem der vorhergehenden Ansprüche, **dadurch gekennzeichnet, dass** der Radialverdichter (3) einen ersten Gasaustritt (31) und einen zweiten Gasaustritt (3p) aufweist.

12. Gasfördervorrichtung (1) nach Anspruch 5, **dadurch gekennzeichnet, dass** innerhalb des Gehäuses (3a) ein Ventil (3m) angeordnet ist, welches derart ausgestaltet und ansteuerbar ist, dass das geförderte Fluid dem ersten und/oder dem zweiten Gasaustritt (31, 3p) zugeleitet ist.

13. Gasfördervorrichtung (1) nach einem der vorhergehenden Ansprüche, **dadurch gekennzeichnet, dass** das Gehäuse (3a) eine in Strömungsrichtung (4e) dem Verdichterrand (3b) vorgelagert angeordnete Öffnung aufweist, durch welche eine Flüssigkeit, insbesondere Wasser einführbar ist.

14. Gasfördervorrichtung (1) nach einem der vorhergehenden Ansprüche, ausgestaltet als Einwegprodukt beziehungsweise als Wegwerfprodukt.

15. Gasfördervorrichtung (1) nach einem der vorhergehenden Ansprüche, umfassend einen Bypass (10), wobei der Bypass (10) eine Fluid leitende Verbindung zwischen der Saugseite (3d) und der Druckseite (3e) des Radialverdichters (3) ausbildet.

16. Gasfördervorrichtung (1) nach Anspruch 15, **dadurch gekennzeichnet, dass** der Bypass (10) ein ansteuerbares Ventil (3m; 11) umfasst.

17. Gasfördervorrichtung (1) nach einem der Ansprüche 15 oder 16, **dadurch gekennzeichnet, dass** das ansteuerbare Ventil (3m) derart im Bereich des Gasaustritts (31) des Radialverdichters (3) angeordnet ist, dass der Gasfluss zumindest teilweise unterbrechbar ist.

18. Gasfördervorrichtung (1) nach Anspruch 17, **dadurch gekennzeichnet, dass** das ansteuerbare Ventil (3m) zungenförmig und schwenkbar ausgestaltet ist.

19. Gasfördervorrichtung (1) nach einem der Ansprüche 15 oder 16, **dadurch gekennzeichnet, dass** der Bypass (10) elastisch ausgestaltet ist, und dass das Ventil als ein mechanisch auf den Bypass (10) einwirkendes Bypassregelventil (11) ausgestaltet ist, um den Innenquerschnitt des Bypasses (10) zu verändern.

20. Gasfördervorrichtung (1) nach Anspruch 19, **dadurch gekennzeichnet, dass** der Bypass (10) einen elastischen Schlauch umfasst.

21. Gasfördervorrichtung (1) nach einem der Ansprüche 19 oder 20, **dadurch gekennzeichnet, dass** das Bypassregelventil (11) ein insbesondere linear angetriebenes Schließteil (11 c) umfasst, welches mechanisch auf den Bypass (10) einwirkt.

22. Gasfördervorrichtung (1) nach einem der Ansprüche 15 bis 21, **dadurch gekennzeichnet, dass** diese eine Antriebsvorrichtung (2) umfasst, welche lösbar mit dem Radialverdichter (3) verbunden ist.

23. Gasfördervorrichtung (1) nach Anspruch 1, wobei das Gehäuse (3a) und der Rotor (3c) derart bezüglich der separaten Antriebsvorrichtung (2) angepasst ausgestaltet sind, dass der Rotor (3c) an die Antriebsvorrichtung (2) koppelbar und von dieser antreibbar ist.

24. Gasfördervorrichtung (1) nach Anspruch 23, **dadurch gekennzeichnet, dass** der Rotor (3c) als Permanentmagnet ausgestaltet ist.

25. Gasfördervorrichtung (1) nach einem der Ansprüche 23 oder 24, **dadurch gekennzeichnet, dass** das Gehäuse (3a) und/oder das Verdichterrad (3b) aus einem Kunststoff besteht.

26. Gasfördervorrichtung (1) nach einem der Ansprüche 23 bis 25, **dadurch gekennzeichnet, dass** ein Bypass (10) eine Fluid leitende Verbindung zwischen einer Saugseite (3d) und einer Druckseite (3e) des Radialverdichters (3) ausbildet.

27. Gasfördervorrichtung (1) nach einem der Ansprüche 23 bis 26, **dadurch gekennzeichnet, dass** der Bypass (10) zumindest abschnittweise aus einem elastischen Material, insbesondere einem Schlauch gebildet ist.

28. Gasfördervorrichtung (1) nach einem der Ansprüche 23 bis 27, **dadurch gekennzeichnet, dass** der der Druckseite (3e) des Radialverdichters (3) nachfolgende, gasleitende Abschnitt (23) aus Kunststoff besteht, und vorzugsweise abschnittweise aus einem elastischen Material, insbesondere einem Schlauch gebildet ist.

29. Gasfördervorrichtung (1) nach einem der Ansprüche 23 bis 28, **dadurch gekennzeichnet, dass** zumindest der Radialverdichter (3) als Einwegprodukt beziehungsweise als Wegwerfprodukt ausgestaltet ist.

30. Gasfördervorrichtung (1) nach einem der Ansprüche 23 bis 29, **dadurch gekennzeichnet, dass** die Gasfördervorrichtung (1) eine Volumenstrommessvorrichtung (5) und/oder eine Druckmessvorrichtung (7) und/oder einen Gasbestimmungssensor (8) und/oder eine Antriebsvorrichtung (2) zum Antrieb des magnetisch aktiven Teils (3c) umfasst, welche mehrfach verwendbar ausgestaltet sind.

31. Gasfördervorrichtung (1) nach einem der Ansprüche 24 bis 30, **dadurch gekennzeichnet, dass** der Radialverdichter (3) und/oder der gasleitende Abschnitt (23) derart ausgestaltet ist, dass eine lösbare Verbindung mit der Volumenstrommessvorrichtung (5) und/oder der Druckmessvorrichtung (7) und/oder dem Gasbestimmungssensor (8) und/oder der Antriebsvorrichtung (2) erstellbar ist.

## Claims

1. A gas conveying device (1) for respiratory and anaesthesia apparatuses, comprising a radial compressor (3) as well as a drive device (2) generating a rotating magnetic field, whereby the radial compressor (3) includes a housing (3a), inside which a compressor impeller (3b) and an associated rotor (3c, 3f, 3g) is arranged, whereby the drive device (2) for the rotor (3c 3f, 3g) is arranged outside the housing (3a),
**characterised in that**
the rotor (3c, 3f, 3g) with the drive device (2) forms an active radial bearing (2c, 3f; 2e, 3g) as a magnetic bearing.

2. The gas conveying device (1) according to claim 1, **characterised in that** there is arranged inside the housing (3a) a step bearing (3r), which supports the compressor impeller (3b) and the rotor (3c) in a rotary manner with respect to the housing (3a).

3. The gas conveying device (1) according to any one of the preceding claims, **characterised in that** the drive device (2) is designed as a motor stator (2d) comprising a motor winding (2b) and a magnetic yoke (2a), so that the motor stator (2d) drives the rotor (3c).

4. The gas conveying device (1) according to claim 1, **characterised in that** the motor stator (2a; 2d) together with the rotor (3c) is designed as a bearing-less motor.

5. The gas conveying device (1) according to any one of the preceding claims, **characterised in that** the rotor (3c) comprises a permanent magnet.

6. The gas conveying device (1) according to any one of claims 1 to 3, **characterised in that** the drive device (2) comprises a rotary magnetic coupling (2g), which can be arranged with respect to the rotor (3c) in such a way that the rotating magnetic coupling (2g) drives the rotor (3c).

7. The gas conveying device (1) according to any one of the preceding claims, **characterised in that** the radial compressor (3) and the drive device (2) are designed so as to be mutually separable.

8. The gas conveying device (1) according to any one of the preceding claims, **characterised in that** the radial compressor (3) has a valve (3m) in the region of the gas outlet (3 1), said valve being designed in particular as an adjustable tongue, in order to interrupt the gas flow at least partially.

9. The gas conveying device (1) according to claim 8, **characterised in that** the valve (3m) is mounted so as to be capable of swivelling and in particular is mounted so as to be mobile about a rotary centre (3o).

10. The gas conveying device (1) according to any one of claims 8 or 9, **characterised in that** the valve (3m) comprises a permanent magnet (3s), and that a suitably adapted drive device is arranged outside the radial compressor (3), in order to move the valve (3m) via the permanent magnet (3s).

11. The gas conveying device (1) according to any one of the preceding claims, **characterised in that** the radial compressor (3) has a first gas outlet (3 1) and a second gas outlet (3p).

12. The gas conveying device (1) according to claim 5, **characterised in that** there is arranged inside the housing (3a) a valve (3m), which is designed and can be controlled in such a way that the conveyed fluid is fed to the first and/or the second gas outlet (3 1, 3p).

13. The gas conveying device (1) according to any one of the preceding claims, **characterised in that** the housing (3a) has an opening arranged upstream - in flow direction (4e) - of the compressor impeller (3b), through which opening a fluid, in particular water, can be introduced.

14. The gas conveying device (1) according to any one of the preceding claims, designed as a non-returnable product, or more precisely as a disposable product.

15. The gas conveying device (1) according to any one of the preceding claims, comprising a bypass (10), whereby the bypass (10) forms a fluid-conveying connection between the suction side (3d) and the pressure side (3e) of the radial compressor (3).

16. The gas conveying device (1) according to claim 15, **characterised in that** the bypass (10) comprises a controllable valve (3m; 11).

17. The gas conveying device (1) according to any one of claims 15 or 16, **characterised in that** the controllable valve (3m) is arranged in the region of the gas outlet (3 1) of the radial compressor (3) in such a way that the gas flow can be interrupted at least partially.

18. The gas conveying device (1) according to claim 17, **characterised in that** the controllable valve (3m) is designed tongue-shaped and capable of swivelling.

19. The gas conveying device (1) according to any one of claims 15 or 16, **characterised in that** the bypass (10) is designed elastic, and that the valve is designed as a bypass control valve (11) acting mechanically on the bypass (10), in order to change the internal cross-section of the bypass (10).

20. The gas conveying device (1) according to claim 19, **characterised in that** the bypass (10) comprises an elastic tube.

21. The gas conveying device (1) according to any one of claims 19 or 20, **characterised in that** the bypass control valve (11) comprises an, in particular, linearly driven closure part (11c), which acts mechanically on the bypass (10).

22. The gas conveying device (1) according to any one of claims 15 to 21, **characterised in that** the latter comprises a drive device (2), which is connected detachably to the radial compressor (3).

23. The gas conveying device (1) according to claim 1, whereby the housing (3a) and the rotor (3c) are designed adapted with respect to the separate drive device (2) in such a way that the rotor (3c) can be coupled to the drive device (2) and driven by the latter.

24. The gas conveying device (1) according to claim 23, **characterised in that** the rotor (3c) is designed as a permanent magnet.

25. The gas conveying device (1) according to any one of claims 23 or 24, **characterised in that** the housing (3a) and/or the compressor impeller (3b) are made from a plastics material.

26. The gas conveying device (1) according to any one of claims 23 to 25, **characterised in that** a bypass (10) forms a fluid-conveying connection between a suction side (3d) and a pressure side (3e) of the radial compressor (3).

27. The gas conveying device (1) according to any one of claims 23 to 26, **characterised in that** the bypass (10) is formed at least in sections from an elastic material, in particular a tube.

28. The gas conveying device (1) according to any one of claims 23 to 27, **characterised in that** the gas-conveying segment (23) following the pressure side (3e) of the radial compressor (3) is made from a plastics material, and is formed preferably in sections from an elastic material, in particular a tube.

29. The gas conveying device (1) according to any one of claims 23 to 28, **characterised in that** at least the radial compressor (3) is designed as a non-returnable product, or more precisely as a disposable product.

30. The gas conveying device (1) according to any one of claims 23 to 29, **characterised in that** the gas conveying device (1) comprises a volume-flow measuring device (5) and/or a pressure measuring device (7) and/or a gas determination sensor (8) and/or a drive device (2) for driving the magnetically active part (3c), which are designed so that they can be used repeatedly.

31. The gas conveying device (1) according to any one of claims 24 to 30, **characterised in that** the radial compressor (3) and/or the gas-conveying segment (23) is designed in such a way that a detachable connection can be made with the volume-flow device (5) and/or the pressure measuring device (7) and/or the gas detection sensor (8) and/or the drive device (2).

## Revendications

1. Dispositif d'alimentation en gaz pour un appareil de ventilation et anesthésie, comprenant un compresseur radial (3) ainsi qu'un dispositif d'entraînement (2) qui génère un champ magnétique tournant, où le compresseur radial (3) comprend un boîtier (3a), à l'intérieur duquel est montée une roue de compresseur (3b) et un rotor (3c, 3f, 3g) connecté à celle-ci, où le dispositif d'entraînement (2) pour le rotor (3c, 3f, 3g) est monté à l'extérieur du boîtier (3a),
**caractérisé en ce que**,
le rotor (3c, 3f, 3g) avec le dispositif d'entraînement (2) forme un palier radial actif (2c, 3f, 2e, 3g) en tant que palier magnétique.

2. Dispositif d'alimentation en gaz (1) selon la revendication 1, **caractérisé en ce qu'**un palier de piste (3r) est prévu à l'intérieur du boîtier (3a), lequel loge la roue de compresseur (3b) et le rotor (3c) pour être tournés par rapport au boîtier (3a).

3. Dispositif d'alimentation en gaz (1) selon l'une des revendications précédentes, **caractérisé en ce que** le dispositif d'entraînement (2) est formé en tant que stator de moteur (2d) comprenant un bobinage moteur (2b) et une clôture de fer (2a), pour que le stator de moteur (2d) entraîne le rotor (3c).

4. Dispositif d'alimentation en gaz (1) selon la revendication 1, **caractérisé en ce que** le stator de moteur (2a, 2g) est formé avec le rotor (3c) en tant que moteur sans palier.

5. Dispositif d'alimentation en gaz (1) selon l'une des revendications précédentes, **caractérisé en ce que** le rotor (3c) comprend un aimant permanent.

6. Dispositif d'alimentation en gaz (1) selon l'une des revendications 1 à 3, **caractérisé en ce que** le dispositif d'entraînement (2) comprend un embrayage magnétique (2g) tournant, qui peut être monté de telle façon par rapport au rotor (3c), que l'embrayage magnétique (2g) tournant entraîne le rotor (3c).

7. Dispositif d'alimentation en gaz (1) selon l'une des revendications précédentes, **caractérisé en ce que** le compresseur radial (3) et le dispositif d'entraînement (2) sont arrangés d'une manière séparable l'un par rapport à l'autre.

8. Dispositif d'alimentation en gaz (1) selon l'une des revendications précédentes, **caractérisé en ce que** le compresseur radial (3) comprend dans la zone de sortie de gaz (31) une vanne (3m), en particulier formée en tant que langue ajustable, pour interrompre au moins partiellement le flux de gaz.

9. Dispositif d'alimentation en gaz (1) selon la revendication 8, **caractérisé en ce que** la vanne (3m) est agencée d'une manière orientable et surtout agencée pour être tournée autour d'un centre de rotation (3o).

10. Dispositif d'alimentation en gaz (1) selon l'une des revendications 8 ou 9, **caractérisé en ce que** la vanne comprend un aimant permanent (3s) et **en ce qu'**un dispositif d'entraînement est prévu à l'extérieur du compresseur radial (3), adapté au dispositif, pour déplacer la vanne (3m) par-dessus de l'aimant permanent (3s).

11. Dispositif d'alimentation en gaz (1) selon l'une des revendications précédentes, **caractérisé en ce que** le compresseur radial (3) comprend une première sortie de gaz (31) et une deuxième sortie de gaz (3p).

12. Dispositif d'alimentation en gaz (1) selon la revendication 5, **caractérisé en ce qu'**une vanne (3m) est montée à l'intérieur du boîtier (3a), qui est agencé d'une telle manière et contrôlable, pour que le liquide convoyé soit amené envers la première et/ou la deuxième sortie de gaz (31, 3p).

13. Dispositif d'alimentation en gaz (1) selon l'une des revendications précédentes, **caractérisé en ce que** le boîtier (3a) comprend une ouverture prévue en amont de la direction du flux (4e) par rapport au bord de compresseur (3b), par la quelle un liquide peut être introduit, en particulier de l'eau.

14. Dispositif d'alimentation en gaz (1) selon l'une des revendications précédentes, formé en tant que produit jetable ou en tant que produit à utiliser une seule fois.

15. Dispositif d'alimentation en gaz (1) selon l'une des revendications précédentes, comprenant une dérivation (10), où la dérivation (10) forme une connexion amenant un liquide entre la partie aspirante (3d) et la partie sous pression (3e) du compresseur radial (3).

16. Dispositif d'alimentation en gaz (1) selon la revendication 15, **caractérisé en ce que** la dérivation (10) comprend une vanne contrôlable (3m; 11).

17. Dispositif d'alimentation en gaz (1) selon l'une des revendications 15 ou 16, **caractérisé en ce que** la vanne contrôlable (3m) est montée de telle manière dans la zone de la sortie de gaz (31) du compresseur radial (3), que le flux de gaz peut au moins partiellement être interrompu.

18. Dispositif d'alimentation en gaz (1) selon la revendication 17, **caractérisé en ce que** la vanne contrôlable (3m) est agencée d'une manière orientable et est en forme d'une langue.

19. Dispositif d'alimentation en gaz (1) selon l'une des revendications 15 ou 16, **caractérisé en ce que** la dérivation (10) est façonnée d'une manière élastique et **en ce que** la vanne est formée en tant que vanne de réglage de dérivation qui peut agir mécaniquement sur la dérivation (10) pour changer la section intérieure de la dérivation (10).

20. Dispositif d'alimentation en gaz (1) selon la revendication 19, **caractérisé en ce que** la dérivation (10) comprend un tuyau élastique.

21. Dispositif d'alimentation en gaz (1) selon l'une des revendications 19 ou 20, **caractérisé en ce que** la vanne réglable de la dérivation (11) comprend en particulier une section de fermeture (11c), déplaçable d'une manière linéaire, qui agit d'une manière mécanique sur la dérivation (10).

22. Dispositif d'alimentation en gaz (1) selon l'une des revendications 15 à 21, **caractérisé en ce qu'**il comprend un dispositif d'entraînement (2) qui est attaché au compresseur radial (3) d'une manière qu'il peut être enlevé.

23. Dispositif d'alimentation en gaz (1) selon la revendication 1, ou le boîtier (3a) et le rotor (3c) sont prévus et agencés d'une telle manière par rapport au dispositif d'entraînement (2), que le rotor (3c) peut être embrayé au dispositif d'entraînement (2) et peut être entraîné par celui-ci.

24. Dispositif d'alimentation en gaz (1) selon le revendication 23, **caractérisé en ce que** le rotor (3c) est arrangé en tant qu'aimant permanent.

25. Dispositif d'alimentation en gaz (1) selon l'une des revendications 23 ou 24, **caractérisé en ce que** le boîtier (3a) et/ou la roue de compresseur (3b) consistent dans un matériel plastique.

26. Dispositif d'alimentation en gaz (1) selon l'une des revendications 23 à 25, **caractérisé en ce** la dérivation (10) forme une connexion amenant du liquide entre la partie d'aspiration (3d) et la partie sous pression (3e) du compresseur radial (3).

27. Dispositif d'alimentation en gaz (1) selon l'une des revendications 23 à 26, **caractérisé en ce que** la dérivation (10) est formé au moins en partie d'un matériel élastique, en particulier d'un tuyau.

28. Dispositif d'alimentation en gaz (1) selon l'une des revendications 23 à 27, **caractérisé en ce que** la partie sous pression (3e) du compresseur radial (3) est suivie par une partie (23) subséquente et fournissant de gaz en matériel plastique, et en particulier formée en partie d'un matériel élastique, en particulier d'un tuyau.

29. Dispositif d'alimentation en gaz (1) selon l'une des revendications 23 à 28, **caractérisé en ce que** le compresseur radial (3) est arrangé en tant que produit jetable ou en tant que produit à utiliser une seule fois.

30. Dispositif d'alimentation en gaz (1) selon l'une des revendications 23 à 29, **caractérisé en ce que** le dispositif d'alimentation en gaz (1) comprend un dispositif de mesure de flux volumétrique (5) et/ou un dispositif de mesure de pression (7) et/ou un détecteur de détermination de gaz (8) et/ou un dispositif d'entraînement (2) pour entraîner la partie magnétique active (3c), qui peut être formé d'une manière utilisable plusieurs fois.

31. Dispositif d'alimentation en gaz (1) selon l'une des revendications 24 à 30, **caractérisé en ce que** le compresseur radial (3) et/ou la partie de gaz (23) sont formés de telle manière, qu'un attachement, qui peut être enlevé, est arrangé avec le dispositif de mesure de flux volumétrique (5) et/ou le dispositif de mesure de pression (7) et/ou le détecteur de détermination de gaz (8) et/ou le dispositif d'entraînement (2).
